(19) Europäisches Patentamt
European Patent Office
Office européen des brevets

(11) **EP 3 472 611 B1**

(12) **EUROPEAN PATENT SPECIFICATION**

(45) Date of publication and mention of the grant of the patent:
**15.05.2024 Bulletin 2024/20**

(21) Application number: **17755261.9**

(22) Date of filing: **16.06.2017**

(51) International Patent Classification (IPC):
***G01N 33/48*** *(2006.01)* ***G01N 33/50*** *(2006.01)*
***C12Q 1/68*** *(2018.01)*

(52) Cooperative Patent Classification (CPC):
**C12Q 1/689; G01N 33/6893;** G01N 2800/04; G01N 2800/06

(86) International application number:
**PCT/IT2017/000119**

(87) International publication number:
**WO 2017/216820 (21.12.2017 Gazette 2017/51)**

(54) **METAGENOMIC METHOD FOR IN VITRO DIAGNOSIS OF GUT DYSBIOSIS**

METHODISCHE METHODE ZUR IN-VITRO-DIAGNOSTIK VON DYSBIOSE

MÉTHODE MÉTAGÉNOMIQUE POUR LE DIAGNOSTIC IN VITRO DE LA DYSBIOSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MD**

(30) Priority: **16.06.2016 IT UA20164448**

(43) Date of publication of application:
**24.04.2019 Bulletin 2019/17**

(73) Proprietor: **Ospedale Pediatrico Bambino Gesù**
**00165 Roma (IT)**

(72) Inventors:
- **PUTIGNANI, Lorenza**
  **00165 Roma (IT)**
- **DEL CHIERICO, Federica**
  **00165 Roma (IT)**

(74) Representative: **Gitto, Serena et al**
**Barzanò & Zanardo Roma S.p.A.**
**Via Piemonte, 26**
**00187 Roma (IT)**

(56) References cited:
**WO-A1-2015/066625 WO-A2-2011/140208**

- **EMILY B. HOLLISTER ET AL: "Structure and function of the healthy pre-adolescent pediatric gut microbiome", MICROBIOME, vol. 3, no. 1, 26 August 2015 (2015-08-26) , XP055339318, DOI: 10.1186/s40168-015-0101-x**
- **SUSANNE MUELLER ET AL: "Differences in Fecal Microbiota in Different European Study Populations in Relation to Age, Gender, and Country: a Cross-Sectional Study", APPLIED AND ENVIRONMENTAL MICROBIOLOGY, AMERICAN SOCIETY FOR MICROBIOLOGY, US, vol. 72, no. 2, February 2006 (2006-02), pages 1027-1033, XP002623954, ISSN: 0099-2240, DOI: 10.1128/AEM.72.2.1027-1033.2006**
- **Gemma Louise Kay ET AL: "Differences in the Faecal Microbiome in Schistosoma haematobium Infected Children vs. Uninfected Children", PLoS neglected tropical diseases, 2015, pages 1-14, XP055339317, United States DOI: 10.1371/journal.pntd.0003861 Retrieved from the Internet: URL:http://journals.plos.org/plosntds/article/file?id=10.1371/journal.pntd.0003861&type=printable**
- **N. O. KAAKOUSH ET AL: "Microbial Dysbiosis in Pediatric Patients with Crohn's Disease", JOURNAL OF CLINICAL MICROBIOLOGY, vol. 50, no. 10, October 2012 (2012-10), pages 3258-3266, XP055237520, US ISSN: 0095-1137, DOI: 10.1128/JCM.01396-12**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- OHAD MANOR ET AL: "Metagenomic evidence for taxonomic dysbiosis and functional imbalance in the gastrointestinal tracts of children with cystic fibrosis", SCIENTIFIC REPORTS, vol. 6, 4 March 2016 (2016-03-04), page 22493, XP055339001, DOI: 10.1038/srep22493
- MIHAI POP ET AL: "Diarrhea in young children from low-income countries leads to large-scale alterations in intestinal microbiota composition", GENOME BIOLOGY, BIOMED CENTRAL LTD., LONDON, GB, vol. 15, no. 6, 27 June 2014 (2014-06-27), pages 1-12, XP021189854, ISSN: 1465-6906, DOI: 10.1186/GB-2014-15-6-R76
- A. HEVIA ET AL: "Intestinal Dysbiosis Associated with Systemic Lupus Erythematosus", MBIO, vol. 5, no. 5, 30 September 2014 (2014-09-30), pages 1-10, XP055280661, DOI: 10.1128/mBio.01548-14

## Description

The present invention concerns a metagenomic method for *in vitro* diagnosis of gut dysbiosis. Particularly, the present invention concerns a metagenomic method for *in vitro* diagnosis of gut dysbiosis able to assign a dysbiosis degree in comparison to healthy subjects.

Gut microbiota is a complex community of microorganisms that live in the human gut. Gut microbiota is generally comparable for individuals in selected groups of population, with recent evidence supporting gut microbiota health associated to a state of eubiosis or dysbiosis, depending on physiological or disease-related conditions, respectively.

Notably, deviations from eubiosis can result in a transient or permanent microbiota imbalance known as dysbiosis, which has been linked to several disorders, including inflammatory bowel disease (IBD), such as Crohn's disease (CD), ulcerative colitis (UC), or irritable bowel syndrome (IBS), obesity, nonalcoholic steatohepatitis, type I and type II diabetes, cystic fibrosis, autoimmune diseases or neurological disorders. Traditionally, evaluation of gut microbiota composition has been based on culture-based techniques and more recently on culture-independent techniques such as high-throughput next-generation sequencing (NGS).

The use of these methods has significantly improved the understanding of the role of gut microbiota in health and disease, especially during pediatric age; for example, small intestinal bacterial overgrowth and altered intestinal microbiota are implicated in subgroups of patients with functional bowel disorders.

Microbial profiling under the host-microbe and microbe-microbe interplays is now one of the most promising laboratory tool to describe symbiosis-dysbiosis shift of gut microbiota (Putignani *et al.,* 2016). Gut microbiota has several metabolic, protective, structural, and mucosal functions. When symbiosis switches to gut dysbiosis, the imbalance involves the liver, adipose tissue, and the immune system (IS), and the gut ecosystem loses many bacterial species altering homeostasis.

Hence, after perturbations, the gut microbiota ecosystem can shift to a state of dysbiosis, in which commensal protective function, structural and histological role, and metabolic activities manifest impaired concerted mechanisms. This can involve overgrowth (blooming) of otherwise underrepresented or potentially harmful bacteria (i.e., pathobionts), induced by intrusion or disappearance of individual members (i.e., invading bacterial strains during maturation of infant gut microbiota); shifts in relative bacterial abundances by *external stimuli;* and mutation or horizontal gene transfer can affect healthy status of the subjects. These alterations influence significantly the overall functionality of microbiota, by enhancing the fitness of certain pathogens or commensal stabilizers.

Some methods for detecting gut microbial composition have been described up to now. In the past, the analysis of bacterial ecosystems was based on the microbial growth on laboratory culture media, but the great limitations of this technique resides in the inability to culture the 80% of stool bacteria (Sekirov et al., 2006) As a consequence, new molecular techniques have been developed. In terms of qualitative measurements of the microbiota, methods such as fingerprinting (denaturing gradient gel electrophoresis), terminal restriction fragment length polymorphism, ribosomal intergenic spacer analysis, and 16S ribosomal RNA sequencing are widely used (Blaut et al., 2002). The new automated massive technologies, based on the 16S ribosomal RNA gene sequencing, present in all prokaryotes, can offer a cost-effective solution for rapid sequencing and identification of all bacterial species of the gut. Metagenomics relates to culture-independent studies of microbial communities to explore microbial consortia that inhabit specific niches in plants or in animal hosts, such as mucosal surfaces and human skin.

For quantitative measurements of gut microbiota bacteria distribution, techniques such as fluorescence *in situ* hybridization, catalyzed reporter deposition-fluorescence *in situ* hybridization, quantitative polymerase chain reaction, and scanning electron microscopy *in situ* hybridization have been used (Peter and Sommaruga, 2008). These methods are able to detect change in total number of microorganisms, change in gut microbiota species, or allow to address the presence or absence of specific bacterial species. However, the estimation of these differences need to be established compared to reference individuals selected amongst healthy subjects.

In recent years, the knowledge regarding species and functional composition of the human intestinal microbiome has increased rapidly, but very little is still known about the composition of microbiome in term of level of normobiosis conditions and inter-individual variability associated to geographical and diet-dependent conditions.

Arumugam and colleagues (Arumugam et al., 2011) characterized variations in the composition of the intestinal microbiota in 39 individuals from four continents by analyzing the fecal metagenome. The authors proposed that the intestinal microbial community could be stratified into three groups, called enterotypes. Each of these three enterotypes is identifiable by the variation in the levels of one of three genera: Bacteroides (enterotype 1), Prevotella (enterotype 2), and Ruminococcus (enterotype 3). Despite the stability of these three major groups, their relative proportions and the species present are highly variable between individuals. Therefore, Siezen and Kleerebezem proposed a new term called "faecotypes" instead of "enterotypes," since it is known that the microbial abundance and composition changes dramatically throughout the gut intestinal tract, and perhaps "enterotypes" may not reflect the microbial composition of the whole intestine (Siezen and Kleerebezem, 2011). Although the intestinal microbiota is stable in adulthood, it undergoes fluctuations during childhood and old age. In children, the type of bacteria colonizing the intestine is defined very early according to the type of delivery and feeding modality (Del Chierico *et al.,* 2015).

It is also known that in elderly individuals, there is a decreasing quantity and diversity of species of *Bacteroides* and *Bifidobacterium* and an increase in facultative anaerobe bacteria. Increase of these bacteria genus is harmful to host since they present high proteolytic activity, which is responsible for putrefaction of large bowel (Woodmansey, 2007).

It is also known that diarrhea in young children from low-income countries leads to large scale alterations in intestinal microbiota composition (Mihai Pop et al. 2014). In addition, methods to establish and restore normal gut microbiota finction of subject in need thereof are known (WO2015066625).

However, at present, there are no studies on gut microbiota which are able to provide a reference microbiota reservoir for the proper description of intestinal eubiosis profiles, to be compared, as reference, to the profiles of patients with disorders and gastrointestinal diseases, in order to detect gut dysbiosis and/or the grade of dysbiosis in term, for example, of mild, moderate and severe dysbiosis. Gut dysbiosis refers to a microbial imbalance inside the intestine in comparison to healthy gut microbiota profiles.

In the light of the above it is therefore apparent the need to provide for new methods for the diagnosis of gut dysbiosis able to overcome the disadvantages of known methods.

According to the present invention, the gut microbiota profiling of healthy subjects has been detected by metagenomics. Particularly, gut microbiota composition (or profiling) has been detected both qualitatively and quantitatively for every taxonomic level, i.e. phylum, family and species. It has been found that gut microbiota composition is independent on gender, however it is dependent on age of the subjects whom the microbiota belongs for all taxonomic levels, i.e. phylum, family and species taxonomic levels.

In addition, it has been found that gut microbiota composition of a healthy subject does not change over time at all taxonomic levels.

On the basis of the above, the present invention provides the essential criteria for setting up a methagenomic method which is surprisingly able to detect every grade of gut dysbiosis of a patient in a significantly statistical way in comparison to a healthy control group. Specifically, gut microbiota composition (or profiling) of a patient can be compared with gut microbiota composition of healthy subjects who are the same or similar age as the patient. A statistically significant difference between gut microbiota of a patient and gut microbiota of healthy subjects is detected at family and species taxonomic levels for every grade of dysbiosis, whereas a statistically significant difference is obtained only in patients with very serious dysbiosis at phylum taxonomic level.

The healthy subjects should be selected preferably among those having overlapping dietary habits (the same or similar), since gut microbiota can be influenced by nutrition patterns and environmental stimuli. For instance, dietary habits depend on geographical area and culture which results in different kinds of diet such as, for example, Mediterranean diet, Japonese diet, Western diet, African diet. Therefore, the healthy subjects should be selected among those having the same kind of diet, with an income of nutrients pretty balanced, resembling a complete omnivore diet, rather than prevalently vegetarian or even vegan.

Preferably, the healthy subjects could be selected among those coming and living in the same geographical area, for instance in the same country or nation, in addition to being selected on the basis of the dietary habits, possibly excluding groups of individuals characterized by highly strict dietary habits.

A specific object of the present invention is therefore a method for providing a gut microbiota reference control tool of healthy subjects for *in vitro* diagnosis of gut dysbiosis index or percentage according to claim 1.

The cluster according to the invention is therefore an homogeneous cluster, i.e. when identified by the Wald's method, it is characterized by multivariate data revealing characteristics of any structure or patterns present (*e.g.* microbiota profiles generating subgroups belonging to the same clustering tree node) (Agresti, A. 2007. An Introduction to Categorical Data Analysis, 2nd ed., New York: John Wiley & Sons. Everitt, B. 2011. Cluster analysis. Chichester, West Sussex, U.K: Wiley. ISBN 9780470749913).

Each cluster can comprise biological samples of at least 10 subjects.

For each cluster, a median value of the operational taxonomic units distribution is obtained for each of said all phyla, families and species, i.e. three median values of the operational taxonomic units distribution are obtained for each cluster.

All phyla, families or species are all phyla, families and species detectable on the basis of the knowledge at the time of detection.

According to the present invention, said clusters are clusters wherein the gut biological samples belong to healthy subjects whose age ranges from 2 years to less than 4 years, from 4 years to less than 7 years, from 7 years to less than 9 years, from 9 years to less than 11 years, from 11 years to less than 13 years, from 13 years to less than 17 years, and/or from 17 years to 70 years.

Thefore, the method of the present invention can be used for in vitro diagnosis of gut dysbiosis index or percentage in pediatric age or childhood as well as in adulthood.

Gut biological samples to be used in the method of the present invention can be faecal samples, gut tissue samples, preferably faecal samples.

According to the present invention, the healthy subjects preferably come from the same Nation.

As mentioned above, gut biological samples to be used according to the present invention are faecal samples, gut tissue samples, preferably faecal samples.

According to the present invention, the healthy subjects preferably come from the same Nation.

The present invention concerns also a method for *in vitro* diagnosis of gut dysbiosis index or percentage according to claim 4.

The dissimilarity index or percentage is calculated by comparing data which refer to the same taxonomic level, i.e. phylum, family or species and then to all phyla, families and species of gut microbiota of the patient compared to controls.

In detail, the dissimilarity index or percentage can be calculated for said all phyla, families and species of gut microbiota of the patient by the formula:

$$Z = (1/2 \times \Sigma(f_{case} - f_{controls})^2)^{1/2}$$

or

$$Z = (1/2 \times \Sigma(f_{case} - f_{controls})^2)^{1/2} \times 100$$

wherein $f_{case}$ is the median value of the operational taxonomic units distribution of said all phyla, families and species of gut microbiota of the patient;

and $f_{controls}$ is the median value of the operational taxonomic units distribution of all phyla, families and species of gut microbiota of the cluster of the gut microbiota reference control tool of healthy subjects as defined above, wherein said cluster is that in which the age of the patient falls in the age range of the healthy subjects of the same cluster.

According to the method of the present invention, the patient preferably comes from the same Nation of the healthy subjects of the control tool as defined above.

The index or percentage varies from 0 to 100 or from 0 to 1: the value 0 means no dissimilarity and the value 100 or 1 means max dissimilarity.

The methods for detecting gut microbiota prevalently qualitatively are well known. For example, fingerprinting (denaturing gradient gel electrophoresis), terminal restriction fragment length polymorphism, ribosomal intergenic spacer analysis, and 16S ribosomal RNA sequencing (Blaut et al.,2002) are known.

Particularly, gut microbiota can be detected by amplifying and pyrosequencing V1-V3 region of 16S ribosomal RNA gene of the microorganisms contained in a gut biological sample according to Ercolini et al, 2012. In a typical gut metagenomic experiment, after DNA extraction from fecal sample, a short segment of the 16S rRNA is amplified. By amplifying and sequencing selected regions within 16S rRNA genes, bacteria can be identified. The identity at phylum, family and species taxonomic level and frequency of bacteria in a sample are determined by assigning reads to known 16S rRNA database sequences via sequence homology. After homology process, however, frequencies of reads and, hence, frequencies of bacteria are assigned by using Quantitative Insights into Microbial Ecology (QIIME 1.8.0, as below reported in detail. Therefore, the method according to the present invention can be a metagenomic method.

The present invention now will be described by an illustrative, but not limitative way, according to preferred embodiments thereof, with particular reference to enclosed drawings, wherein:

**Figure 1.** - Clustering of controls by Wald's method at L2 taxon level - 3 groups ( curly brackets from I to III) 6 groups (curly brackets from A to F).

**Figure 2.** - Clustering of controls by Wald's method at L5 taxon level - 3 groups (curly brackets from I to III) 6 groups (curly brackets from A to F).

**Figure 3** - Clustering of controls by Wald's method at L6 taxon level - 3 groups (curly brackets from I to III) 6 groups (curly brackets from A to F).

**EXAMPLE 1:** ***Study of microbiota profiling***

**Introductory materials and methods for microbiota profiling generation.**

**1. Relative abundances of OTUs calculated by metagenomics.**

Three and one stool sample was collected and processed from each patient and each reference subject, respectively. Genomic DNA was isolated from the entire set of 96 samples, using the QIAamp DNA Stool Mini Kit (Qiagen, Germany). The V1-V3 region of 16S ribosomal RNA (rRNA) locus was amplified for next pyrosequencing step on a 454-Junior Genome Sequencer (Roche 454 Life Sciences, Branford, USA). Reads were analyzed by Quantitative Insights into Microbial Ecology (QIIME, v.1.8.0), grouped into operational taxonomic units (OTUs) at a sequence similarity level of 97% by PyNAST for taxonomic assignment, and aligned by UCLUST for OTUs matching against Greengenes database (v. 13.8).

*Genomic DNA extraction.* Genomic DNA was extracted from all faecal samples. Stools were resuspended into 1.5 ml PBS, homogenized by vortexing for 2 min and centrifuged at 20,800×g. After supernatant removal, pellet was resuspended into 500 μl of PBS added by 500 μl of Beads/PBS (1 mg/μl, w/v) (Glass Beads, acid-washed SigmaAldrich). The 1:1 mixture was homogenized by vortexing for 2 min and centrifuged at 5200×*g* for 1 min. The supernatant was collected, and treated for one freeze-thaw cycle (-20°C/70°C) for 20 min each step. After centrifugation at 5200×*g* for 5 min, the supernatant was subjected to QIAamp DNA Stool Mini Kit (Qiagen, Germany) extraction, according to manufacturer's instructions. DNA was eluted into 50 μl purified $H_2O$ (Genedia, Italy) and its yield quantified using a NanoDrop ND-1000 spectrophotometer (NanoDrop Technologies, Wilmington, DE). DNA was adjusted to 10 ng/μl concentration and used as template for successful 16S Metagenomic 454 Sequencing Analyses.

*Amplicon library preparation and pyrosequencing.* Gut microbiome was investigated by pyrosequencing V1-V3 regions of 16S rRNA gene (amplicon size 520 bp), on a GS Junior platform (454 Life Sciences, Roche Diagnostics, Italy), according to the pipeline described elsewhere (Ercolini *et al,* 2012). In a typical gut metagenomic experiment, after DNA extraction from fecal sample, a short segment of the 16S rRNA is amplified. By amplifying and sequencing selected regions within 16S rRNA genes, bacteria can be identified. The identity at phylum, family and species taxonomic level and frequency of bacteria in a sample are determined by assigning reads to known 16S rRNA database sequences via sequence homology.

For the metagenomics analysis needs:

QIAAMP DNA STOOL MINI KIT (Qiagen) for DNA extraction from fecal samples; Fast Start Hi-Fi PCR system dNTP Pack (Roche diagnostics) for 16S rRNA amplification;

EmPCR Kit Oil and Breaking Kit, EmPCR Kit EmPCR Reagents (Lib-L), EmPCR Bead Recovery Reagents, Sequencing Kit Reagents and Enzymes, Sequencing Kit Packing Beads and Supplement CB, Sequencing Kit Buffers, PicoTiterPlate Kit (Roche diagnostics) for pyrosequencing reactions.

*Bioinformatics.* A first result filtering was performed using the 454 Amplicon signal processing; sequences were then analyzed by using Quantitative Insights into Microbial Ecology (QIIME 1.8.0) software (Caporaso et al., 2010). In order to guarantee a higher level of accuracy in terms of Operational Taxonomic Units (OTUs) detection, after demultiplexing, reads with an average quality score lower than 25, shorter than 300 bp, and with an ambiguous base calling were excluded from the analysis.

Sequences that passed the quality filter were denoised (Reeder *et al.,* 2010) and singletons were excluded. The OTUs defined by a 97% of similarity were picked using the uclust method (Edgar et al., 2010) and the representative sequences were submitted to PyNAST, for the sequence aligment the used method was UCLUST and the database for OTUs matching was greengenes (v 13.8). The last step consisted in building an OTU table with the absolute abundance of each OTU across all samples, followed by the taxonomic assignment: 6 levels of deep taxonomy (from kingdom to species), unassigned OTUs and unspecified levels were considered.

Ecological diversity for each sample was assessed by: *i*) number of OTUs obtained from each samples; *ii*) Shannon index, giving the entropy information of the observed OUT abundances and account for both richness and eveness; *iii*) Chaol metric estimating species richness; *iv)* phylogenetic distance (PD_whole_tree) to assess quantitative measure of phylogenetic diversity; *v*) observed species metric, counting unique OTUs found in the sample; *vi*) Good's coverage, measuring the percentage of the total species represented in a sample. The β-diversity, representing the comparison of microbial communities based on their dissimilar composition, was calculated by unweighted and weighted UNIFRAC and Bray-Curtis algorithms. The α and β diversity and the Kruskal Wallis test were performed by QIIME software, using "alpha_rarefaction.py, beta_diversity_through_py, group_significance.py" scripts. Furthermore, to measure the robustness of the results a jackknifing analysis was performed. To measure the robustness of this data a jackknifing analysis was performed on data subsets, and the resulting Unweighted Pair Group Method with Arithmetic (UPGMA) tree was compared with the entire data set tree (jackknifed_beta_diversity.py -i otus/otu_table.txt -t otus/rep_set.tre -m Fasting_Map.txt -o wf_jack -e). This process was repeated with many random subsets of data (the 75% of the smallest number of sequences for samples), and the tree nodes that prove more consistent across jackknifed datasets were deemed more robust.

**1.1 Criteria for patients/controls' pairs selection.**

An operational database, including microbiota OTUs distribution data from 96 faecal samples, 79 from controls e 17 from 6 patients' samples (3 samples collected for each patient, except in one case) was built up accordingly to age stratification groups (2-3; 4-6; 7-8;9-10; 11-12; 13-16 years of age) and for each L2 (phylum)-L5 (Family)-L6 (species) taxonomic levels **(Table 1).**

**Table 1.** Correlation dataset between patient and control groups

| N | #SampleID | Age | Gender | Group | patient | N | #SampleID | Age | Gender | Group | patient |
|---|---|---|---|---|---|---|---|---|---|---|---|
| **1** | **N.11.9** | 2 | | 2_3 | | **42** | **N.04.1** | 9 | m | 9_10 | |
| **2** | **N.11.1** | 2 | m | 2_3 | | **43** | **N.04.2** | 9 | f | 9_10 | |
| **3** | **N.11.2** | 2 | m | 2_3 | | **44** | **N.04.3** | 9 | f | 9_10 | |
| **4** | **N.11.3** | 2 | m | 2_3 | | **45** | **N.04.4** | 9 | m | 9_10 | |
| **5** | **N.11.4** | 2 | f | 2_3 | | **46** | **N.04.5** | 9 | m | 9_10 | |
| **6** | **N.11.8** | 2 | m | 2_3 | | **47** | **N.04.6** | 9 | f | 9_10 | |
| **7** | **N11.5** | 2 | f | 2_3 | | **48** | **N.04.7** | 9 | f | 9_10 | |
| **8** | **N11.6** | 2 | m | 2_3 | Ver | **49** | **N.04.8** | 9 | m | 9_10 | |
| **9** | **N11.7** | 2 | f | 2_3 | | **50** | **N.03.03** | 10 | m | 9_10 | Pasc |
| **10** | **N.10.1** | 3 | m | 2_3 | | **51** | **N.03.1** | 10 | f | 9_10 | |
| **11** | **N.10.2** | 3 | f | 2_3 | | **52** | **N.03.2** | 10 | m | 9_10 | |
| **12** | **N.10.4** | 3 | m | 2_3 | | **53** | **N.03.4** | 10 | m | 9_10 | |
| **13** | **N.10.5** | 3 | f | 2_3 | | **54** | **N.03.5** | 10 | f | 9_10 | |
| **14** | **N.10.6** | 3 | m | 2_3 | | **55** | **N.03.6** | 10 | f | 9_10 | |
| **15** | **N10.3** | 3 | m | 2_3 | | **56** | **N.03.7** | 10 | f | 9_10 | |
| **16** | **N09.6** | 4 | m | 4_6 | | **57** | **N.03.8** | 10 | m | 9_10 | |
| **17** | **N09.7** | 4 | f | 4_6 | | **58** | **N.02.1** | 11 | f | 11_12 | |
| **18** | **N09.9** | 4 | f | 4_6 | | **59** | **N.02.2** | 11 | f | 11_12 | |
| **19** | **N.09.4** | 4 | m | 4_6 | | **60** | **N.02.3** | 11 | f | 11_12 | |
| **20** | **N08.1** | 5 | m | 4_6 | | **61** | **N.02.4** | 11 | f | 11_12 | |
| **21** | **N08.5** | 5 | f | 4_6 | | **62** | **N.02.5** | 11 | f | 11_12 | |
| **22** | **N07.5** | 6 | f | 4_6 | | **63** | **N.02.6** | 11 | m | 11_12 | |
| **23** | **N.07.3** | 6 | f | 4_6 | | **64** | **N.02.7** | 11 | m | 11_12 | |
| **24** | **N.07.4** | 6 | m | 4_6 | | **65** | **N.02.8** | 11 | f | 11_12 | |
| **25** | **N.07.6** | 6 | f | 4_6 | | **66** | **N.01.1** | 12 | m | 11_12 | |
| **26** | **N.06.1** | 7 | f | 7_8 | | **67** | **N.01.2** | 12 | f | 11_12 | |
| **27** | **N.06.2** | 7 | m | 7_8 | | **68** | **N.00.1** | 13 | m | 13_16 | |
| **28** | **N.06.4** | 7 | m | 7_8 | | **69** | **N.00.2** | 13 | f | 13_16 | |
| **29** | **N.06.5** | 7 | m | 7_8 | | **70** | **N.00.3** | 13 | m | 13_16 | |
| **30** | **N.06.7** | 7 | m | 7_8 | | **71** | **N.00.4** | 13 | f | 13_16 | |
| **31** | **N.06.8** | 7 | f | 7_8 | | **72** | **N.00.5** | 13 | f | 13_16 | |
| **32** | **N06.6** | 7 | m | 7_8 | | **73** | **N.00.6** | 13 | f | 13_16 | |
| **33** | **N.05.1** | 8 | m | 7_8 | | **74** | **N.98.3** | 14 | f | 13_16 | Cag Per Spar |
| **34** | **N.05.2** | 8 | f | 7_8 | Deg | **75** | **N.99.1** | 14 | m | 13_16 | |
| **35** | **N.05.3** | 8 | m | 7_8 | | **76** | **N.99.2** | 14 | f | 13_16 | |
| **36** | **N.05.4** | 8 | m | 7_8 | | **77** | **N.98.1** | 15 | m | 13_16 | |
| **37** | **N.05.5** | 8 | m | 7_8 | | **78** | **N.98.2** | 15 | f | 13_16 | |
| **38** | **N.05.6** | 8 | m | 7_8 | | **79** | **N.97.01** | 16 | f | 13_16 | |
| **39** | **N.05.7** | 8 | m | 7_8 | | | | | | | |
| **40** | **N.05.8** | 8 | m | 7_8 | | | | | | | |
| **41** | **N.05.9** | 8 | f | 7_8 | | | | | | | |

## 2 Question n° 1: Can the controls be divided into groups?

### 2.1 Statistical methods.

A hierarchical cluster analysis with Wald's method has been performed in order to group controls into a limited number of homogeneous clusters. The cluster is characterized by multivariate data revealing characteristics of any structure or patterns present (e.g., microbiota profiles generating subgroups belonging to the same clustering tree node) (see Everitt, B. 2011. Cluster analysis. Chichester, West Sussex, U.K: Wiley. ISBN 9780470749913. Agresti, A. 2007. An Introduction to Categorical Data Analysis, 2nd ed., New York: John Wiley & Sons. The number of clusters was chosen by dendrogram computation. The *null hypothesis* of independence between gender and clusters and age groups (2-3, 4-6, 7-8, 9-10, 11-12, 13-16) and cluster was tested by chi square independence test. P-values were computed both analytically and by re-sampling (see Agresti, 2007).

Controls are clustered using L2, L5 and L6 taxon levels.

### 2.2 Main results.

Clusters were independent on gender and dependent on age groups for all taxon levels. Therefore, at each taxon level it was meaningful to group controls by age and not by gender.

#### a) Clustering at L2 taxon level.

Main results: clustering was independent on gender and dependent on age group. So it is meaningful to group controls by age and not by gender.

**Figure 1.** - Clustering of controls by Wald's method at L2 taxon level - 3 groups (curly brackets from I to III) 6 groups (curly brackets from A to F).

Both with 3 and 6 clusters gender and groups resulted statistically independent as p-values were larger than 5%. Both with 3 and 6 clusters age and groups resulted statistically dependent as p-values were smaller than 1% (see **Table 2).**

**Table 2** - Chi square test of independence between clusters and age and cluster and gender at L2 taxon level.

| Categories | Chi sq. Test | p. value (exact) | p. value (approx) |
|---|---|---|---|
| Age_groups vs. 3 clusters | 139.4 | < 2.2*10^-16 | < 2.2*10^-16 |
| Age_groups vs. 6 clusters | 277.24 | < 2.2*10^-16 | < 2.2*10^-16 |
| Gender vs. 3 clusters | 7.37 | 0.1173 | 0.0821 |
| Gender vs. 6 clusters | 10.91 | 0.3645 | 0.3585 |
| p-value approximation is computed by 9999 resamplings | | | |

#### b) Clustering at L5 taxon level.

***Main results:*** clustering was independent on gender and dependent on age group. Therefore, it was meaningful to group controls by age and not by gender.

**Figure 2.** - Clustering of controls by Wald's method at L5 taxon level - 3 groups (curly brackets from I to III) 6 groups (curly brackets from A to F).

Both with 3 and 6 clusters gender and groups resulted statistically independent as p-values were larger than 5%. Both with 3 and 6 clusters age and groups resulted statistically dependent as p-values were smaller than 1% (see **Table 3).**

**Table 3** - Chi square test of independence between clusters and age and cluster and gender at L5 taxon level

| Categories | Chi sq. Test | p. value (exact) | p. value (approx) |
|---|---|---|---|
| Age_groups *vs.* 3 clusters | 144.85 | < 2.2*10^-16 | < 2.2*10^-16 |
| Age_groups *vs.* 6 clusters | 353.92 | < 2.2*10^-16 | < 2.2*10^-16 |
| Gender *vs.* 3 clusters | 4.97 | 0.2908 | 0.2983 |

(continued)

| Categories | Chi sq. Test | p. value (exact) | p. value (approx) |
|---|---|---|---|
| Gender *vs.* 6 clusters | 10.16 | 0.4265 | 0.4187 |
| p-value approximation is computed by 9999 resamplings | | | |

**c) Clustering at L6 taxon level.**

Main results: clustering was independent on gender and dependent on age group. Therefore, it was meaningful to group controls by age and not by gender.

**Figure 3** - Clustering of controls by Wald's method at L6 taxon level - 3 groups (curly brackets from I to III) 6 groups (curly brackets from A to F).

Both with 3 and 6 clusters gender and groups resulted statistically independent as p-values were larger than 5%. Both with 3 and 6 clusters age and groups resulted statistically dependent as p-values were smaller than 1% (see **Table 4).**

**Table 4** - Chi square test of independence between clusters and age and cluster and gender at L6 taxon level

| Categories | Chi sq. Test | p. value (exact) | p. value (approx) |
|---|---|---|---|
| Age_groups *vs.* 3 clusters | 120.38 | $< 2.2*10^{\wedge-16}$ | $< 2.2*10^{\wedge-16}$ |
| Age_groups *vs.* 6 clusters | 244.32 | $< 2.2*10^{\wedge-16}$ | $< 2.2*10^{\wedge-16}$ |
| Gender vs. 3 clusters | 6.99 | 0.14 | 0.08 |
| Gender vs. 6 clusters | 9.11 | 0.52 | 0.51 |
| p-value approximation is computed by 9999 resamplings | | | |

**3. Question n° 2: Do the samples from each** patient **change over time?**

**Statistical methods.**

Three samples were collected from each patient at three different times (three consecutive days). By using the Kruskal-Wallis rank sum test (see Kruskal and Wallis, 1952) the null hypothesis that the median of the three samples is the same against the alternative hypothesis that they differed in at least one sample has been tested.

The test was performed on each patient and at L2, L5 and L6 taxon levels.

**Main result:** the medians of samples from all patients were the same at any time, *i.e.* they did not change over time at all taxon levels. All p-values were greatly higher than 10% (see **Table 5).**

**Table 5** - Kruskal - Wallis test on all patients at L2, L5, L6 taxon levels

| Patient | Taxon | Kruskal Wallis chi sqare | df[1] | p-value |
|---|---|---|---|---|
| n° 1 - Cag | L2 | 0.0099 | 2 | 0.9951 |
| | L5 | 0.9621 | 2 | 0.6181 |
| | L6 | 22.025 | 2 | 0.3325 |
| n°2 - Deg | L2 | 0.0016 | 1 | 0.9678 |
| | L5 | 0.025 | 1 | 0.8743 |
| | L6 | 0.0347 | 1 | 0.8521 |
| n°3 - Pas | L2 | 0.3553 | 2 | 0.8372 |
| | L5 | 0.6616 | 2 | 0.7183 |
| | L6 | 0.8649 | 2 | 0.6489 |

(continued)

| Patient | Taxon | Kruskal Wallis chi sqare | df[1] | p-value |
|---|---|---|---|---|
| n°4 - Per | L2 | 0.0744 | 2 | 0.9635 |
| | L5 | 0.4667 | 2 | 0.7919 |
| | L6 | 13.238 | 2 | 0.5159 |
| n °5 - Spar | L2 | 0.0829 | 2 | 0.9594 |
| | L5 | 0.0463 | 2 | 0.9791 |
| | L6 | 0.2370 | 2 | 0.8882 |
| n°6 - Ver | L2 | 0.5957 | 2 | 0.7424 |
| | L5 | 0.4141 | 2 | 0.8130 |
| | L6 | 0.0282 | 2 | 0.9860 |
| [1]Degree of freedom | | | | |

**4. Question n° 3: Comparison of OTUs distributions between each patient and controls within the same age group**

**Statistical methods.**

We compared the average of each patient's samples (OTUs distribution) and the average of samples of controls from the same age group. By Kruskal-Wallis rank sum test (see Kruskal and Wallis, 1952) we tested the null hypothesis that the medians of the two samples are the same against the alternative hypothesis that they differ at L2, L5 and L6 taxon levels.

**Main results:** as shown in **Table 6,** the difference between cases and controls was not statistically significant at L2 taxon level (p-values are larger than 10% in all patients). Such difference was statistically significant at L5 and L6 taxon level (p-values are smaller than 1% in all patients).

**Table 6** - Kruskal - Wallis test on each patient vs. controls in the same age group at L2, L5, L6 taxon levels

| Patient | Taxon | Kruskal Wallis chi sqare | p-value |
|---|---|---|---|
| n° 1 - Cag | L2 | 1.93 | 0.16 |
| | L5 | 19.62 | $9.43*10^{-6}$ |
| | L6 | 22.74 | $1.84*10^{-6}$ |
| n°2 - Deg | L2 | 2.56 | 0.11 |
| | L5 | 24.01 | $9.56*10^{-7}$ |
| | L6 | 35.04 | $3.23*10^{-9}$ |
| n°3 - Pas | L2 | 2.11 | 0.14 |
| | L5 | 2.08 | $5.51*10^{-7}$ |
| | L6 | 40.21 | $2.28*10^{-7}$ |
| n°4 - Per | L2 | 0.70 | 0.40 |
| | L5 | 6.12 | 0.01 |
| | L6 | 9.52 | $2*10^{-3}$ |
| n°5 - Spar | L2 | 0.83 | 0.36 |
| | L5 | 6.25 | 0.01 |
| | L6 | 3.27 | 0.07 |

(continued)

| Patient | Taxon | Kruskal Wallis chi sqare | p-value |
|---|---|---|---|
| n°6 - Ver | L2 | 1.21 | 0.27 |
| | L5 | 22.48 | $2.13*10^{\wedge-6}$ |
| | L6 | 26.04 | $3.34*10^{\wedge-7}$ |

## 5. Question n°4: Dysbiosis: dissimilarity measure between cases and controls

### Statistical methods

As in Leti (1983), we used the percentage quadratic dissimilarity index

$$Z = (1/2*\Sigma(f_{case} - f_{controls})^\wedge 2)^{\wedge 1/2}$$

where $f_{case}$ is the OTUs distribution in a patient and $f_{controls}$ is the OTUs distribution among controls in the same age group. This index varied between 0 and 1 and can be expressed in percentage. The value 0 means no dissimilarity and the value 1 means max dissimilarity. Therefore, this index is suitable to be used as a measure of dysbiosis.

We computed it only at L5 and L6 taxon levels and not at L2, because in previous section it has been proved that OTUs distributions are statistically different between each case and controls within the same age group at L5 and L6 taxon levels and not at L2 level.

### Main results

1. Patient Cag showed a dissimilarity degree versus controls at 35% (L6) - 36% (L5) of maximum dissimilarity.
2. Patient Deg showed a dissimilarity degree versus controls at 38% (L6) - 40% (L5) of maximum dissimilarity.
3. Patient Pas showed a dissimilarity degree versus controls at 26% (L5) - 29% (L6) of maximum dissimilarity.
4. Patient Per showed a dissimilarity degree versus controls at 30% (L5) - 31% (L6) of maximum dissimilarity.
5. Patient Spar showed a dissimilarity degree versus controls at 10% (L5) - 28% (L6) of maximum dissimilarity.
6. Patient Ver showed a dissimilarity degree versus controls at 29% (L5) -36% (L6) of maximum dissimilarity.

**Table 7** - Dysbiosis or dissimilarity index between OTUs distribution in each patient vs. OTUs distribution in controls in the same age group at L5 and L6 taxon levels

| Patient | Taxon level | Dysbiosis index |
|---|---|---|
| n°1 - Cag | L5 | 0.3661 |
| | L6 | 0.3573 |
| n°2 - Deg | L5 | 0.4001 |
| | L6 | 0.3842 |
| n°3 - Pas | L5 | 0.2698 |
| | L6 | 0.2928 |
| n°4 - Per | L5 | 0.3019 |
| | L6 | 0.3184 |
| n°5 - Spar | L5 | 0.1074 |
| | L6 | 0.2795 |
| n°6 - Ver | L5 | 0.2911 |
| | L6 | 0.3601 |

**EXAMPLE 2**: ***Extension of microbiota profiling from childhood to adulthood***

The method of comparing the patient microbiota profile to the healthy reference groups (CTRLs) was extended from the childhood age to the adulthood. With this aim, besides the groups of 2-3; 4-6; 7-8; 9-10; 11-12; 13-16 years of age, a group of controls from 17-70 years was added to the CTRLs groups, consistently with what recently described (N Engl J Med 375; 24, December 15, 2016) and even improved in the range 12-16. Also in this group median values of OTUs distribution were calcultated for each L2 (phylum)-L5 (Family)-L6 (species) taxonomic levels (data not shown). Accordingly, the dissimilarity percentage was calculated for the adult range in a way to apply the dysbiosis computation also to faecal samples collected by adult patients.

**References**

1. Putignani L, Del Chierico F, Vernocchi P, Cicala M, Cucchiara S, Dallapiccola B; Dysbiotrack Study Group. Gut Microbiota Dysbiosis as Risk and Premorbid Factors of IBD and IBS Along the Childhood-Adulthood Transition. Inflamm Bowel Dis. 2016 Feb;22(2):487-504.
2. Sekirov I, Russell SL, Antunes LC, Finlay BB. Gut microbiota in health and disease. Physiol Rev. 2010;90:859-904;
3. Dethlefsen L, Eckburg PB, Bik EM, Reiman DA. Assembly of the human intestinal microbiota. Trends Ecol Evol. 2006;21:517-523
4. Blaut M, Collins MD, Welling GW, Dore J, Van LJ, de Vos W. Molecular biological methods for studying the gut microbiota: The EU human gut flora project. Br J Nutr. 2002;87(Suppl 2):S203-211;
5. McCartney AL. Application of molecular biological methods for studying probiotics and the gut flora. Br J Nutr. 2002;88(Suppl 1):S29-S37
6. Peter H, Sommaruga R. An evaluation of methods to study the gut bacterial community composition of freshwater zooplankton. J Plankton Res. 2008;30:997-1006
7. Arumugam M., Raes J., Pelletier E., et al. Enterotypes of the human gut microbiome. Nature. 2011;473(7346):174-180. doi: 10.1038/nature09944
8. Siezen R. J., Kleerebezem M. The human gut microbiome: are we our enterotypes? Microbial Biotechnology. 2011;4(5):550-553. doi: 10.1111 /j.1751-7915.2011.00290.x.
9. Del Chierico F, Vernocchi P, Petrucca A, Paci P, Fuentes S, Praticò G, Capuani G, Masotti A, Reddel S, Russo A, Vallone C, Salvatori G, Buffone E, Signore F, Rigon G, Dotta A, Miccheli A, de Vos WM, Dallapiccola B, Putignani L). Phylogenetic and Metabolic Tracking of Gut Microbiota during Perinatal Development. PLoS One. 2015 Sep 2;10(9):e0137347. doi: 10.1371/journal.pone.0137347. eCollection 2015.
10. Woodmansey E. J. Intestinal bacteria and ageing. Journal of Applied Microbiology. 2007;102(5):1178-1186. doi: 10.1111/j.1365-2672.2007.03400.x
11. Ercolini D, De Filippis F, La Storia A, Iacono M. "Remake" by high-throughput sequencing of the microbiota involved in the production of water buffalo mozzarella cheese. Appl. Environ. Microbiol. 2012;78:8142-8145.
12. Caporaso, JG, Kuczynski, J, Stombaugh, J, Bittinger, K, Bushman, FD, Costello, EK, et al.. QIIME allows analysis of high-throughput community sequencing data. Nat. Methods 2010;7, 335-336.
13. Reeder J, Knight R. Rapidly denoising pyrosequencing amplicon reads by exploiting rank-abundance distributions. Nat. Methods. 2010;7:668-669.
14. Edgar RC. Search and clustering orders of magnitude faster than BLAST. Bioinforma. Oxf. Engl. 2010;26:2460-2461.
15. Agresti, A. 2007. An Introduction to Categorical Data Analysis, 2nd ed., New York: John Wiley & Sons.
16. Everitt, B. 2011. Cluster analysis. Chichester, West Sussex, U.K: Wiley. ISBN 9780470749913.
17. Kruskal, WH and Wallis, A. 1952. "Use of ranks in one-criterion variance analysis". Journal of the American Statistical Association. 47: 583-621.
18. Leti, G. (1983), Elementi di statistica descrittiva, Il Mulino, Milano.
19. Mihai Pop et al., "Diarrhea in young children from low-income countries leads to large scale alterations in intestinal microbiota composition", Genome Biology, Biomed Central Ltd, London, GB, vol. 15, no. 6, 27 June 2014, pages 1-12.


**Claims**

**1.** Method for providing a gut microbiota reference control tool of healthy subjects for *in vitro* diagnosis of gut dysbiosis index or dysbiosis percentage, said method comprising or consisting of:

a) clustering gut biological samples of healthy subjects in clusters wherein the gut biological samples belong to healthy subjects whose age ranges from 2 years to less than 4 years, from 4 years to less than 7 years, from 7 years to less than 9 years, from 9 years to less than 11 years, from 11 years to less than 13 years, from 13 years to less than 17 years, and/or from 17 years to 70 years;
b) detecting by metagenomics the identity and frequency of all phyla, families and species of gut microbiota in the gut biological samples of each of said healthy subjects of each of said clusters; and
c) calculating the median values of the operational taxonomic units distribution for each of said clusters.

**2.** Method according to claim 1, wherein the gut biological samples are chosen from the group consisting of faecal samples, gut tissue samples, preferably faecal samples.

**3.** Method according to anyone of claims 1-2, wherein the healthy subjects come from the same Nation.

**4.** Method for *in vitro* diagnosis of gut dysbiosis index or dysbiosis percentage comprising or consisting of:

a) detecting by metagenomics the identity and frequency of all detectable phyla, families and species of gut microbiota in more than two, preferably three, gut biological samples of a patient which are collected in consecutive days;
b) calculating the median values of operational taxonomic units distribution of said all detectable phyla, families and species of said gut biological samples of the patient;
c) calculating the dissimilarity index or percentage of the median values of the operational taxonomic units distributions of gut microbiota of the patient in comparison with the median values of the operational taxonomic units distribution of a cluster of the gut microbiota reference control tool of healthy subjects obtained by the method as defined in anyone of the claims 1-3, wherein said cluster is that in which the age of the patient falls in the age range of the healthy subjects of the same cluster, and using said dissimilarity index or percentage as a measure of gut dysbiosis index or percentage.

**5.** Method according to claim 4, wherein the dissimilarity index or percentage is calculated for said all phyla, families and species of gut microbiota of the patient by the formula:

$$Z = (1/2 \times \Sigma(f_{case} - f_{controls})^2)^{1/2}$$

or

$$Z = (1/2 \times \Sigma(f_{case} - f_{controls})^2)^{1/2} \times 100$$

wherein $f_{case}$ is the median value of the operational taxonomic units distribution of said all phyla, families and species of gut microbiota of the patient;
and $f_{controls}$ is the median value of the operational taxonomic units distribution of all phyla, families and species of gut microbiota of the cluster of the gut microbiota reference control tool of healthy subjects
obtained by the method as defined in anyone of the claims 1-3, wherein said cluster is that in which the age of the patient falls in the age range of the healthy subjects of the same cluster.

**6.** Method according to anyone of claims 1-6, wherein the patient comes from the same Nation of the healthy subjects of the control tool of healthy subjects obtained by the method as defined in anyone of the claims 1-3.

## Patentansprüche

**1.** Verfahren zur Bereitstellung eines Referenzkontrollinstruments für die Darmmikrobiota gesunder Probanden zur *in-vitro* Diagnose des DarmDysbiose-Index oder des Dysbiose-Prozentsatzes, wobei das Verfahren Folgendes umfasst oder daraus besteht:

a) Gruppieren von biologischen Darmproben gesunder Probanden in Clustern, wobei die biologischen Darmproben gesunden Probanden gehören, deren Alter im Bereich von 2 Jahren bis weniger als 4 Jahren, von 4 Jahren bis weniger als 7 Jahren, von 7 Jahren bis weniger als 9 Jahren liegt, von 9 Jahren bis unter 11 Jahren, von 11 Jahren bis unter 13 Jahren, von 13 Jahren bis unter 17 Jahren und/oder von 17 Jahren bis 70 Jahren;
b) Nachweis der Identität und Häufigkeit aller Stämme, Familien und Arten von Darmmikrobiota durch Metagenomik in den biologischen Darmproben jedes der gesunden Probanden jedes der Cluster; und
c) Berechnen der Medianwerte der Verteilung der operativen taxonomischen Einheiten für jeden der Cluster.

**2.** Verfahren nach Anspruch 1, wobei die biologischen Darmproben aus der Gruppe bestehend aus Stuhlproben, Darmgewebeproben, vorzugsweise Stuhlproben, ausgewählt werden.

**3.** Verfahren nach einem der Ansprüche 1-2, wobei die gesunden Probanden aus derselben Nation stammen.

**4.** Verfahren zur *in vitro* Diagnose des Darm Dysbiose Index oder des Dysbiose Prozentsatzes, umfassend oder bestehend aus:

a) Nachweis der Identität und Häufigkeit aller nachweisbaren Stämme, Familien und Arten der Darmmikrobiota durch Metagenomik in mehr als zwei, vorzugsweise drei, biologischen Darmproben eines Patienten, die an aufeinanderfolgenden Tagen entnommen werden;
b) Berechnen der Medianwerte der operativen taxonomischen Einheitenverteilung aller nachweisbaren Phyla, Familien und Arten der biologischen Darmproben des Patienten;
c) Berechnen des Unähnlichkeitsindex oder Prozentsatzes der Medianwerte der operativen taxonomischen Einheitenverteilungen der Darmmikrobiota des Patienten im Vergleich zu den Medianwerten der operativen taxonomischen Einheitenverteilung eines Clusters des Referenzkontrollinstruments der Darmmikrobiota gesunder Probanden durch das Verfahren nach einem der Ansprüche 1-3, wobei der Cluster derjenige ist, in dem das Alter des Patienten in den Altersbereich der gesunden Probanden desselben Clusters fällt, und Verwendung des Unähnlichkeitsindex oder Prozentsatzes als Maß des Darmdysbiose-Index oder Prozentsatzes.

**5.** Verfahren nach Anspruch 4, wobei der Unähnlichkeitsindex oder prozentsatz für alle Stämme, Familien und Arten der Darmmikrobiota des Patienten nach der Formel berechnet wird:

$$Z = (1/2 \times \sum(f_{case} - f_{controls})^2)^{1/2}$$

oder

$$Z = (1/2 \times \sum(f_{case} - f_{controls})^2)^{1/2} \times 100$$

wobei $f_{case}$ der Medianwert der operativen taxonomischen Einheitenverteilung aller Phyla, Familien und Arten der Darmmikrobiota des Patienten ist;
und $f_{controls}$ ist der Medianwert der operativen taxonomischen Einheitenverteilung aller Phyla, Familien und Arten von Darmmikrobiota des Clusters des Darmmikrobiota-Referenzkontrollinstruments gesunder Probanden, der durch das in einem der Ansprüche 1-3 definierte Verfahren erhalten wurde, wobei die Gruppe diejenige ist, bei der das Alter des Patienten in den Altersbereich der gesunden Probanden derselben Gruppe fällt.

**6.** Verfahren nach einem der Ansprüche 1-6, wobei der Patient aus derselben Nation stammt wie die gesunden Probanden des Kontrollinstruments für gesunde Probanden, das durch das Verfahren gemäß einem der Ansprüche 1-3 erhalten wurde.

## Revendications

**1.** Procédé pour fournir un outil de contrôle de référence du microbiote intestinal de sujets sains pour le diagnostic *in vitro* de l'indice de dysbiose intestinale ou du pourcentage de dysbiose, ledit procédé comprenant ou consistant à:

a) regrouper des échantillons biologiques intestinaux de sujets sains en grappes dans lesquels les échantillons biologiques intestinaux appartiennent à des sujets sains dont l'âge est compris entre 2 ans et moins de 4 ans, entre 4 ans et moins de 7 ans, entre 7 ans et moins de 9 ans, de 9 ans à moins de 11 ans, de 11 ans à moins de 13 ans, de 13 ans à moins de 17 ans, et/ou de 17 ans à 70 ans;
b) détecter par métagénomique l'identité et la fréquence de tous les phylums, familles et espèces du microbiote intestinal dans les échantillons biologiques intestinaux de chacun desdits sujets sains de chacun desdits clusters; et
c) calculer les valeurs médianes de la distribution des unités taxonomiques opérationnelles pour chacun desdits groupes.

**2.** Procédé selon la revendication 1, **caractérisé en ce que** les échantillons biologiques intestinaux sont choisis dans le groupe constitué des échantillons fécaux, des échantillons de tissus intestinaux, de préférence des échantillons fécaux.

**3.** Procédé selon l'une quelconque des revendications 1-2, dans lequel les sujets sains proviennent de la même nation.

**4.** Procédé de diagnostic *in vitro* de l'indice de dysbiose intestinale ou du pourcentage de dysbiose comprenant ou consistant en:

a) détecter par métagénomique l'identité et la fréquence de tous les phylums, familles et espèces détectables du microbiote intestinal dans plus de deux, de préférence trois, échantillons biologiques intestinaux d'un patient qui sont collectés au cours de jours consécutifs;
b) calculer les valeurs médianes de la distribution des unités taxonomiques opérationnelles de tous les phylums, familles et espèces détectables des échantillons biologiques intestinaux du patient;
c) calculer l'indice de dissimilarité ou le pourcentage des valeurs médianes des distributions d'unités taxonomiques opérationnelles du microbiote intestinal du patient par rapport aux valeurs médianes de la distribution des unités taxonomiques opérationnelles d'un groupe de l'outil de contrôle de référence du microbiote intestinal de sujets sains obtenu par le procédé tel que défini dans l'une quelconque des revendications 1-3, dans lequel ledit groupe est celui dans lequel l'âge du patient se situe dans la tranche d'âge des sujets sains du même groupe, et en utilisant ledit indice ou pourcentage de dissimilarité comme mesure de l'indice ou du pourcentage de dysbiose intestinale.

**5.** Procédé selon la revendication 4, dans laquelle l'indice ou pourcentage de dissimilarité est calculé pour lesdits tous les phylums, familles et espèces du microbiote intestinal du patient par la formule:

$$Z = (1/2 \times \sum(f_{case} - f_{controls})^2)^{1/2}$$

ou

$$Z = (1/2 \times \sum(f_{case} - f_{controls})^2)^{1/2} \times 100$$

où $f_{case}$ est la valeur médiane de la distribution des unités taxonomiques opérationnelles de tous les phylums, familles et espèces du microbiote intestinal du patient;
et $f_{controls}$ est la valeur médiane de la distribution des unités taxonomiques opérationnelles de tous les phylums, familles et espèces du microbiote intestinal du groupe de l'outil de contrôle de référence du microbiote intestinal de sujets sains obtenu par le procédé tel que défini dans l'une quelconque des revendications 1-3, ledit groupe étant celui dans lequel l'âge du patient se situe dans la tranche d'âge des sujets sains du même groupe.

**6.** Procédé selon l'une quelconque des revendications 1-6, dans lequel le patient provient de la même nation que les sujets sains de l'outil de contrôle des sujets sains obtenu par le procédé tel que défini dans l'une quelconque des revendications 1-3.

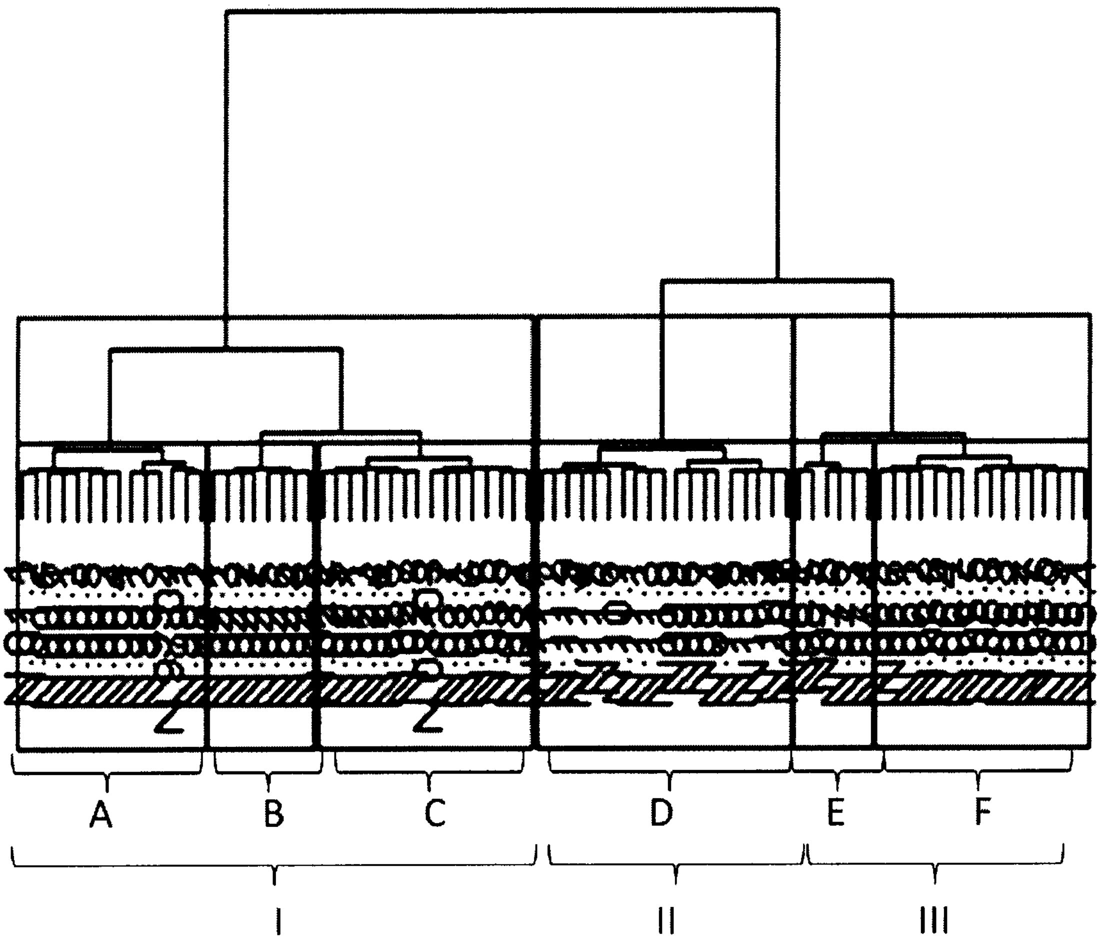


Fig. 1

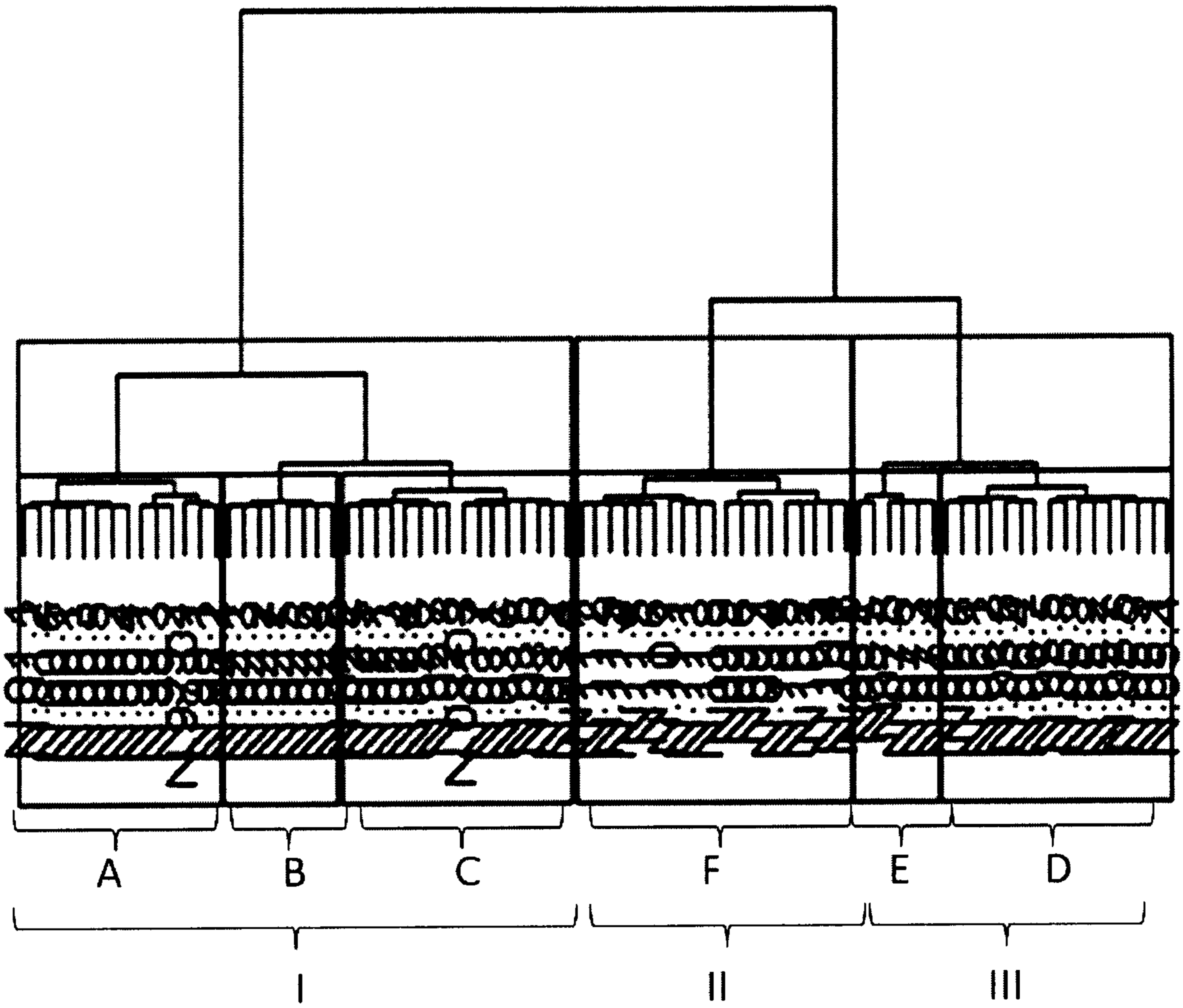


Fig. 2

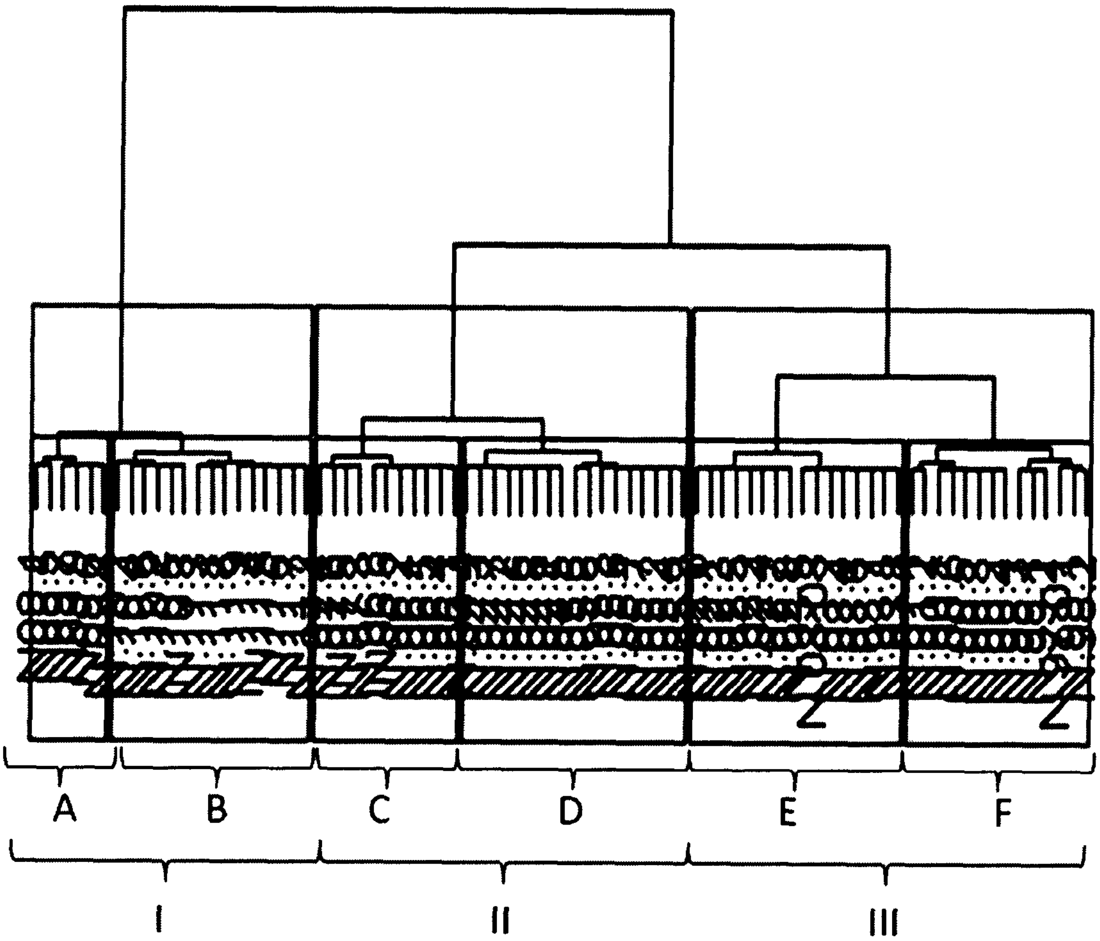


Fig. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2015066625 A


### Non-patent literature cited in the description


- **AGRESTI, A.** An Introduction to Categorical Data Analysis. John Wiley & Sons, 2007
- **EVERITT, B.** Cluster analysis. Wiley, 2011
- **EVERITT, B.** Cluster analysis. Wiley, 2011
- *N Engl J Med,* 15 December 2016, vol. 375, 24
- **PUTIGNANI L ; DEL CHIERICO F ; VERNOCCHI P ; CICALA M ; CUCCHIARA S ; DALLAPICCOLA B.** Dysbiotrack Study Group. Gut Microbiota Dysbiosis as Risk and Premorbid Factors of IBD and IBS Along the Childhood-Adulthood Transition. *Inflamm Bowel Dis.,* February 2016, vol. 22 (2), 487-504
- **SEKIROV I ; RUSSELL SL ; ANTUNES LC ; FINLAY BB.** Gut microbiota in health and disease. *Physiol Rev.,* 2010, vol. 90, 859-904
- **DETHLEFSEN L ; ECKBURG PB ; BIK EM ; REIMAN DA.** Assembly of the human intestinal microbiota. *Trends Ecol Evol,* 2006, vol. 21, 517-523
- **BLAUT M ; COLLINS MD ; WELLING GW ; DORE J ; VAN LJ ; DE VOS W.** Molecular biological methods for studying the gut microbiota: The EU human gut flora project. *Br J Nutr.,* 2002, vol. 87 (2), S203-211
- **MCCARTNEY AL.** Application of molecular biological methods for studying probiotics and the gut flora. *Br J Nutr.,* 2002, vol. 88 (1), S29-S37
- **PETER H ; SOMMARUGA R.** An evaluation of methods to study the gut bacterial community composition of freshwater zooplankton. *J Plankton Res,* 2008, vol. 30, 997-1006
- **ARUMUGAM M. ; RAES J. ; PELLETIER E. et al.** Enterotypes of the human gut microbiome. *Nature,* 2011, vol. 473 (7346), 174-180
- **SIEZEN R. J. ; KLEEREBEZEM M.** The human gut microbiome: are we our enterotypes?. *Microbial Biotechnology,* 2011, vol. 4 (5), 550-553
- **DEL CHIERICO F ; VERNOCCHI P ; PETRUCCA A ; PACI P ; FUENTES S ; PRATICÒ G ; CAPUANI G ; MASOTTI A ; REDDEL S ; RUSSO A.** Phylogenetic and Metabolic Tracking of Gut Microbiota during Perinatal Development. *PLoS One,* 02 September 2015, vol. 10 (9), e0137347
- **WOODMANSEY E. J.** Intestinal bacteria and ageing. *Journal of Applied Microbiology,* 2007, vol. 102 (5), 1178-1186
- **ERCOLINI D ; DE FILIPPIS F ; LA STORIA A ; LACONO M.** Remake'' by high-throughput sequencing of the microbiota involved in the production of water buffalo mozzarella cheese. *Appl. Environ. Microbiol,* 2012, vol. 78, 8142-8145
- **CAPORASO, JG ; KUCZYNSKI, J ; STOMBAUGH, J ; BITTINGER, K ; BUSHMAN, FD ; COSTELLO, EK et al.** QIIME allows analysis of high-throughput community sequencing data. *Nat. Methods,* 2010, vol. 7, 335-336
- **REEDER J ; KNIGHT R.** Rapidly denoising pyrosequencing amplicon reads by exploiting rank-abundance distributions. *Nat. Methods.,* 2010, vol. 7, 668-669
- **EDGAR RC.** Search and clustering orders of magnitude faster than BLAST. *Bioinforma. Oxf. Engl,* 2010, vol. 26, 2460-2461
- **KRUSKAL, WH ; WALLIS, A.** Use of ranks in one-criterion variance analysis. *Journal of the American Statistical Association,* 1952, vol. 47, 583-621
- **LETI, G.** Elementi di statistica descrittiva. *Mulino,* 1983, vol. II
- Diarrhea in young children from low-income countries leads to large scale alterations in intestinal microbiota composition. **MIHAI POP et al.** Genome Biology. Biomed Central Ltd, 27 June 2014, vol. 15, 1-12